# EUROPÄISCHE PATENTSCHRIFT

(11) **EP 2 243 024 B1**
(45) Veröffentlichungstag und Bekanntmachung des Hinweises auf die Patenterteilung: **03.04.2019**
(21) Anmeldenummer: 09710961.5
(22) Anmeldetag: 09.02.2009
(51) Int. Cl.: G01N 27/403, G01N 33/487, G01N 33/543

(54) **EINRICHTUNG UND VERFAHREN ZUM NACHWEIS VON FLÜSSIGKEITEN ODER SUBSTANZEN AUS FLÜSSIGKEITEN**
APPARATUS AND METHOD FOR THE DETECTION OF LIQUIDS OR SUBSTANCES FROM LIQUIDS
DISPOSITIF ET PROCÉDÉ POUR DÉTECTER DES LIQUIDES OU DES SUBSTANCES DE LIQUIDES

(30) Priorität: 15.02.2008 DE 102008009185
(43) Veröffentlichungstag der Anmeldung: 27.10.2010
(73) Patentinhaber: Boehringer Ingelheim Vetmedica GmbH, 55216 Ingelheim am Rhein (DE)
(72) Erfinder: GUMBRECHT, Walter, 91074 Herzogenaurach (DE); PAULICKA, Peter, 91056 Erlangen (DE); STANZEL, Manfred, 91056 Erlangen (DE)
(74) Vertreter: Von Rohr Patentanwälte Partnerschaft mbB
(86) Internationale Anmeldenummer: PCT/EP2009/051440
(87) Internationale Veröffentlichungsnummer: WO 2009/101048

(56) Entgegenhaltungen:
- WO-A-01/19505
- WO-A-94/08236
- WO-A-96/32635
- WO-A-02/073153
- DE-A1- 10 126 341
- DE-A1-102006 046 776
- DE-C1- 10 058 394

## Beschreibung

Die vorliegende Erfindung bezieht sich auf eine Einrichtung und ein Verfahren zum Nachweis von Flüssigkeiten oder Substanzen aus Flüssigkeiten in räumlich verschiedenen Reaktionsbereichen. Eine entsprechende Einrichtung sowie ein Verfahren zu deren Betrieb gehen aus der DE 10058394 C1 hervor.

In der biochemischen Analytik werden verstärkt Sensor-Arrays mit z.B. elektrochemischen oder optischen Sensoren eingesetzt, um schnell und kostengünstig die Zusammensetzung von Flüssigkeiten wie z.B. Blut oder Urin auf bestimmte Substanzen hin zu testen. So können über Sensor-Arrays mit Sensoren, welche mit verschiedenen DNA-Fängermolekülen beschichtet sind, DNA-Analysen ausgeführt werden. Urin-Proben von Probanden können auf Krankheitserreger hin untersucht werden, indem in Sensor-Arrays Sensoren mit Antikörpern beschichteten sind, und das spezifische Anbinden der Krankheitserreger an die entsprechenden Antikörper gemessen wird. Eine bevorzugte Messmethode ist dabei die elektrochemische Messung von Stoffumsatz, welcher nur an spezifisch gebundenen Molekülen auftritt.

Um eine gute Handhabung der Sensor-Arrays zu erreichen, zielen neuere Entwicklungen darauf ab, diese in kleine, kompakte, tragbare Systeme wie z.B. Chipkarten zu integrieren. Diese enthalten einen Chip mit darauf integriertem Sensor-Array, mikrofluidische Kanäle für den Flüssigkeitstransport zum und vom Chip weg, Reaktionskammern für Reaktionen wie z.B. Aufschluss von Blut und darauffolgende Polymerase-Kettenreaktion (Polymerase Chain Reaction, PCR), sowie Kammern mit chemischen Reagenzien bevorzugt in trockener Form. Genormte Chipkarten sind aus Kunststoff aufgebaut und können in Analysegeräte einfach eingebracht werden. Zuvor werden die vom Patienten entnommene, zu untersuchende Flüssigkeit auf- oder in die Chipkarte eingebracht, die Trockenreagenzien gelöst und für den Nachweis nötige Reaktionen ausgelöst. Bei Einführung der Chipkarte in das Analysengerät werden die Sensoren auf dem Chip über elektrische Kontakte auf der Chipkarte durch das Analysengerät aktiviert und können geregelt bzw. gesteuert biochemische Reaktionen messen sowie die Ergebnisse der Messung an das Analysengerät in Form von optischen oder elektrischen Signalen ausgeben. Das Ergebnis kann dann durch das Analysengerät, nach Auswertung durch eine rechnergestützte Einheit über einen Monitor oder andere Anzeigeinstrumente ausgegeben werden.

Um eine erhöhte Messgenauigkeit und eine geringere Fehlerrate bei der Messung zu erhalten, ist aus dem Stand der Technik wie z.B. der DE 100 58 394 C1 ein Verfahren und eine zugehörige Anordnung bekannt, bei dem/der die Sensoren des Sensor-Arrays bei der Messung so voneinander getrennt werden, dass keine Flüssigkeit von einem Sensor zum anderen strömen kann. Die Sensoren des Sensor-Arrays sind auf einer planaren Oberfläche angeordnet mit Wänden zwischen den einzelnen Sensoren, wobei die Wände aus der Oberfläche herausragen. Parallel zur planaren Oberfläche des Sensor-Arrays ist beabstandet zu den Wänden ein Gehäuseoberteil angeordnet, welches genügend Abstand zu der Oberfläche und den Wänden aufweist, um Flüssigkeit zwischen der Oberfläche und dem Gehäuseoberteil strömen zu lassen. Vor der Messung wird mit Hilfe eines Stempels das Gehäuseoberteil in Richtung Oberfläche mit Wänden gedrückt, so dass die Wände und das Gehäuseoberteil in engem Kontakt stehen.

Das Strömen der Flüssigkeit ist unterbunden, und über den Sensoren sind abgeschlossene Reaktionsräume, begrenzt durch die planare Oberfläche, die Wände und das Gehäuseoberteil, entstanden. Die Flüssigkeit, welche in den Reaktionsräumen eingeschlossen ist, kann nun ohne Flüssigkeitsaustausch zwischen verschiedenen Reaktionsräumen untersucht werden. Die Messung der Sensoren findet mit Hilfe der abgeschlossenen Reaktionsräume unabhängig voneinander statt.

Das im Stand der Technik z.B. gemäß der DE 100 58 394 C1 beschriebene Verfahren führt zu einer komplizierten Anordnung mit Gehäuseoberteil und Stempel, wobei die Wände zwischen den Sensoren genauen Abmessungen entsprechen müssen. Diese genauen Abmessungen sind in der Praxis schwer zu realisieren:
Ragen die Wände zu wenig aus der planaren Oberfläche heraus, in welcher die Sensoren angeordnet sind, so entstehen zu kleine Reaktionsräume mit zu wenig Flüssigkeitsmenge, um eine aussagekräftige Messung durchzuführen. Das Gehäuseoberteil kann unter Umständen, bei hohem Stempeldruck auf den Sensoren aufsitzen, womit die Messung verfälscht wird.

Sind die Wände zu groß dimensioniert, so müssen die Sensoren in einem größeren Abstand voneinander angeordnet werden, und die nötige Flüssigkeitsmenge zum Befüllen der Anordnung steigt. Damit fällt die Empfindlichkeit der Messung geringer aus und die Zahl der zur Messung zur Verfügung stehenden Sensoren sinkt bei gleicher Fläche, da diese größer dimensioniert werden müssen um eine ausreichende Messgenauigkeit zu erreichen.

Die WO 96/32635 A beschreibt ein Verfahren, wobei ein räumlicher Bereich einer Schicht auf einem porösen Substrat definiert wird, indem ein definiertes Volumen des Substrats, z.B. in einem ringförmigen Bereich, in der Regel irreversibel, zusammengedrückt wird. Der zusammengedrückte Bereich des Substrats ist für einen Materialtransport undurchlässig.

Aufgabe der Erfindung ist es, eine im Vergleich zum Stand der Technik einfachere und kostengünstigere Einrichtung sowie Verfahren und deren Anwendung zum Nachweis von Flüssigkeiten oder Substanzen in Flüssigkeiten anzugeben. Insbesondere ist es Aufgabe der Erfindung, die Abhängigkeit der Messgenauigkeit von den Fehlertoleranzen der Fertigung der Wände zwischen den Sensoren zu reduzieren. Ein einfacherer Aufbau mit weniger Fehlermöglichkeiten soll Kosten bei der Herstellung der Einrichtung sparen und die Messgenauigkeit erhöhen.

Die angegebene Aufgabe wird bezüglich der Einrichtung mit den Merkmalen des Anspruchs 1 oder bezüglich des Verfahrens mit den Merkmalen des Anspruchs 9 gelöst.

Vorteilhafte Ausgestaltungen der erfindungsgemäßen Einrichtung und des Verfahrens gehen aus den jeweils zugeordneten abhängigen Unteransprüchen hervor. Dabei können die Merkmale der nebengeordneten Ansprüche mit Merkmalen eines jeweils zugeordneten Unteranspruchs oder vorzugsweise auch mit Merkmalen mehrerer zugeordneter Unteransprüche kombiniert werden.

Die erfindungsgemäße Einrichtung zum Nachweis von Flüssigkeiten oder Substanzen aus Flüssigkeiten in räumlich verschiedenen Reaktionsbereichen bzw. Reaktionsräumen umfasst ein Sensor-Array, welches aus wenigstens zwei Sensoren besteht, und wenigstens eine Membran, welche auf dem Sensor-Array angeordnet ist und permeable oder semipermeable, poröse Eigenschaften aufweist. Die mit dieser Ausgestaltung der Einrichtung verbundenen Vorteile sind insbesondere in ihrem einfachen und kostengünstigen, reproduzierbaren Aufbau bei dennoch hinreichender Messgenauigkeit zu sehen.

Insbesondere umfasst die erfindungsgemäße Einrichtung wenigstens eine Membran, bei welcher abhängig von einer Zug- und/oder Druckkraft die Permeabilität einstellbar ist. Dies ist insbesondere bei Membranen der Fall, welche aus Nitrocellulose bestehen oder Nitrocellulose enthalten und/oder eine Porengröße im Bereich von 0,1 bis 100 Mikrometer Durchmesser aufweisen, besonders bevorzugt im Bereich von 1 bis 10 Mikrometer Durchmesser.

In einer bevorzugten Ausführungsform der erfindungsgemäßen Einrichtung enthält das Sensor-Array elektrochemische Sensoren mit Elektroden, insbesondere Mikro-Elektroden, oder ist daraus aufgebaut ist. Die wenigstens zwei Sensoren sind planar auf einer Oberfläche angeordnet. Besonders bevorzugt sind die Sensoren durch Seitenwände, insbesondere ringförmige Seitenwände, räumlich voneinander teilweise oder vollständig getrennt, insbesondere durch Seitenwände in Form geschlossener Ringe.
Eine alternative Ausführungsform sieht vor, dass die Sensoren in Vertiefungen, insbesondere in kreisförmigen Vertiefungen, angeordnet sind.

Um eine hohe Empfindlichkeit der Messung mit den Sensoren zu erreichen, ist es von Vorteil, dass die Membran formschlüssig auf der Oberfläche angeordnet ist, insbesondere planar ohne Knicke in der Membran.

Das Verfahren zum Nachweis von Flüssigkeiten oder Substanzen aus Flüssigkeiten in räumlich verschiedenen Reaktionsbereichen bzw. Reaktionsräumen, umfasst die Schritte
- Befüllen wenigstens einer permeablen oder semipermeablen Membran, welche auf einem Sensor-Array angeordnet ist, mit einer zu analysierenden Flüssigkeit, und
- Mechanisches Verformen der wenigstens einen permeablen oder semipermeablen Membran derart, dass deren Permeabilität eingestellt wird, und
- Messen von Wechselwirkungen der zu analysierenden Flüssigkeit oder von Substanzen aus der Flüssigkeit mit Sensoren des Sensor-Arrays. Die einzelnen Schritte des Verfahrens können gleichzeitig oder nacheinander durchgeführt werden.

In einer bevorzugten Ausführungsform des Verfahrens nach der Erfindung erfolgt die mechanische Verformung der wenigstens einen permeablen oder semipermeablen Membran durch eine Druckkraft, welche flächig auf eine Hauptoberfläche der Membran ausgeübt wird. Die Druckkraft kann zu einem Zusammenpressen der Membran führen. Dies bewirkt insbesondere eine Verkleinerung der Porenvolumina. Bei abgeschlossenen Reaktionsräumen nimmt das Volumen des für die Reaktion zur Verfügung stehenden Raumes ab und somit nimmt bei gleich verlaufender Reaktion die Konzentration an Reaktionsprodukt zu. Die Empfindlichkeit der Messanordnung wird somit erhöht.

Durch das mechanische Verformen der wenigstens einen permeablen oder semipermeablen Membran kann eine vollständige Unterbindung eines Flüssigkeitsaustausches zwischen unterschiedlichen Bereichen der Membran erfolgen, welche benachbart zu räumlich getrennten Sensoren angeordnet sind. Dies erfolgt insbesondere dann, wenn Seitenwände, insbesondere ringförmige Seitenwände um die Sensoren angeordnet, bei Druckeinwirkung auf die Membran im Bereich der Seitenwände zu einer kleineren Porengröße der Membran im Vergleich zur Porengröße im Bereich der Sensoren führen. Durch die Bildung von abgeschlossenen Reaktionsräumen jeweils für jeden Sensor wird ein "übersprechen", d.h. die Beeinflussung des Messergebnisses in einem Reaktionsraum durch Reaktionen in einem benachbarten Reaktionsraum, verringert bzw. vollständig unterbunden.

In einer besonders bevorzugten Ausführungsform des Verfahrens der Erfindung erfolgt das Befüllen der wenigstens einen permeablen oder semipermeablen Membran mit zu analysierender Flüssigkeit durch vollsaugen der Membran mit der Flüssigkeit aufgrund von Kapillarkräften und/oder durch Druckunterschiede innerhalb der Flüssigkeit.
Das Befüllen kann aber auch durch Verdrängen eines Gases aus der Membran durch die Flüssigkeit erfolgen und/oder durch Strömen, insbesondere kontinuierliches Strömen, der Flüssigkeit durch die Membran.

Weiterhin erfolgt in einer besonders bevorzugten Ausführungsform des Verfahrens das Messen von Wechselwirkungen der zu analysierenden Flüssigkeit oder von Substanzen aus der Flüssigkeit mit Sensoren des Sensor-Arrays über elektrochemische Messungen, insbesondere Voltammetrische- und/oder Chronoamperometrische- und/oder Coulometrische- und/oder Impedanz-Messungen. Von Vorteil ist dabei, wenn während der Messung zwischen räumlich verschiedenen Reaktionsbereichen, welche an die Sensoren direkt angrenzen, kein die Messung störender Flüssigkeitsaustausch und/oder Substanzaustausch, insbesondere überhaupt kein Flüssigkeitsaustausch und/oder Substanzaustausch, stattfindet.

Die Verwendung der zuvor beschriebenen Einrichtung und/oder des Verfahrens umfasst die Verwendung in einer Chipkarte, insbesondere in einer Einweg-Chipkarte, mit einem Chip, z.B. einem CMOS-Chip mit einem Sensor-Array, insbesondere mit elektrochemischen Sensoren, wobei der Chip insbesondere in einer polymeren Vergussmasse, wie z.B. Plastik eingegossen ist und die Membran insbesondere planar, besonders bevorzugt ohne Knicke den Chip abdeckt und mit den Sensoren des Chips in direktem Kontakt steht.

Dabei kann die Chipkarte in ein Auslesegerät, insbesondere in ein Handheld-Auslesegerät, eingesetzt werden, welches insbesondere eine Spannungs- und/oder Stromquelle, ein Display, eine digitale Schnittstelle für den Chip, eine Signalverarbeitungseinheit, einen elektrischen, besonders bevorzugt einen thermostatisierten elektrischen Abgriff, einen Stempel-Aktuator und/oder Reagenz-Aktuator enthält.

Wird ein Druck vom Auslesegerät auf die Chipkarte ausgeübt, insbesondere über einen Stempel flächig, so wird die auf dem Chip liegende Membran so komprimiert, das mit zu analysierender Flüssigkeit gefüllte Bereiche der Membran über unterschiedlichen Sensoren untereinander keine Flüssigkeit austauschen. Damit sind abgeschlossene Reaktionsbereiche in der Membran über den Sensoren ausgebildet. Aufgrund des wassersaugenden Charakters der Membran ist jedoch eine elektrische Leitfähigkeit im Inneren der Membran zumindest teilweise noch gegeben.

Die Verwendung der zuvor beschriebenen Einrichtung und/oder des Verfahrens schließt eine Verwendung in einem immunologischen Schnelltest und/oder in einem Bluttest und/oder in einer DNA-Analyse und/oder in einem Antikörpertest und/oder in einem Peptidtest mit ein.

Der Erfindung liegt allgemein die Idee zu Grunde, dass eine poröse Membran auf dem Sensor-Array aufgebracht ist, über welche der Flüssigkeitstransport entlang der Oberfläche, über die Sensoren hinweg, stattfindet. Durch Zusammenpressen der Membran wird der Flüssigkeitstransport unterbunden und es entstehen über den Sensoren abgeschlossene Reaktionsräume. Dies wird vorrangig durch Erhöhungen wie Wände zwischen den Sensoren bewirkt, welche die Membran bei Druck im Bereich der Wände stärker komprimieren als im Bereich der Sensoren. Dadurch werden im Bereich der Wände die Poren der Membran geschlossen und ein Flüssigkeitstransport in diesem Bereich unterbunden. Im Bereich der Sensoren werden die Poren nur verkleinert und dadurch entstehen geschlossene Reaktionsräume mit verkleinerter Porengröße und geschlossenen Poren im Randbereich der Reaktionsräume. Ein Flüssigkeitstransport in den Reaktionsräumen zu den Sensoren hin ist über die verkleinerten Poren möglich, während ein Flüssigkeitstransport zwischen benachbarten bzw. räumlich unterschiedlichen Reaktionsräumen durch die geschlossenen Poren unterbunden ist. Störungen während der Messung durch Flüssigkeitsaustausch zwischen benachbarten Reaktionsräumen werden verhindert, Austausch von Reaktionsprodukten zwischen Reaktionsräumen unterbunden und die Messgenauigkeit wird erhöht.

Durch die weiche Struktur der Membran, d.h. ihre Kompressionsfähigkeit, ist die Anordnung gegenüber Schwankungen in der Wandhöhe zwischen den Sensoren relativ unempfindlich. Bei Wänden, welche etwas höher sind als ihre Nachbarwände, wird die Membran stärker komprimiert. Eine irreversible Beschädigung der Wände erfolgt jedoch nicht, zumindest solange der Druck einen kritischen Wert nicht übersteigt und die Wände nicht durch die Membran hindurchgedrückt werden. Im Gegensatz dazu ist bei der im Stand der Technik bekannten Anordnung, bei welcher ein steifer Stempel auf die Wände aufsetzt, keine Toleranz der Wandhöhen möglich. Bei der aus dem Stand der Technik bekannten Anordnung wird ein Flüssigkeitsaustausch zwischen allen benachbarten Reaktionsräumen nur unterbunden, wenn der inkompressible Stempel schlüssig mit allen Wänden gleichzeitig in Kontakt steht. Sind Wände höher als andere, dann steht der Stempel mit diesen in Kontakt, mit den niedrigeren Wänden jedoch nicht, und ein Flüssigkeitsaustausch über die niedrigeren Wände ist weiterhin möglich. Bei Erhöhung des Drucks, um eine vollständige Unterbindung des Flüssigkeitsaustausches zwischen benachbarten Reaktionsräumen zu erreichen, können die Wände irreversibel beschädigt werden.

Für das erfindungsgemäße Verfahren und die erfindungsgemäßen Verwendungen ergeben sich die vorstehend erwähnten, mit der erfindungsgemäßen Vorrichtung verbundenen Vorteile.

Bevorzugte Ausführungsformen der Erfindung mit vorteilhaften Weiterbildungen gemäß den Merkmalen der abhängigen Ansprüche werden nachfolgend anhand der folgenden Figuren näher erläutert, ohne jedoch darauf beschränkt zu sein.

Es zeigen:
- Fig. 1: in zwei Teilfiguren A und B eine schematische Darstellung eines Sensor-Arrays nach dem Stand der Technik, mit zu den Sensoren zugeordneten Reaktionsräumen und einem steifen Stempel zum Abdichten der Reaktionsräume gegenüber Flüssigkeitsaustausch, vor und während einer Messung,
- Fig. 2: in drei Teilfiguren C bis E eine schematische Darstellung eines Sensor-Arrays einer erfindungsgemäßen Einrichtung mit Sensoren, welche durch Wände 8 voneinander räumlich abgetrennt sind, und mit einer formschlüssig auf der Oberfläche liegenden Membran, vor und nach Befüllen mit Flüssigkeit und nach dem Zusammendrücken während der Messung,
- Fig. 3: eine schematische Darstellung der Vorder- und Rückseite eines Steckmoduls einer erfindungsgemäßen Einrichtung für immunologische Schnelltests,
- Fig. 4: eine schematische Darstellung eines Querschnitts durch das in Fig. 3 gezeigte Steckmodul,
- Fig. 5: eine vereinfachte Darstellung des in Fig. 4 gezeigten Querschnitts durch das Steckmodul mit Lateral/Vertikal-Flussanordnung und CMOS-Chip 27.

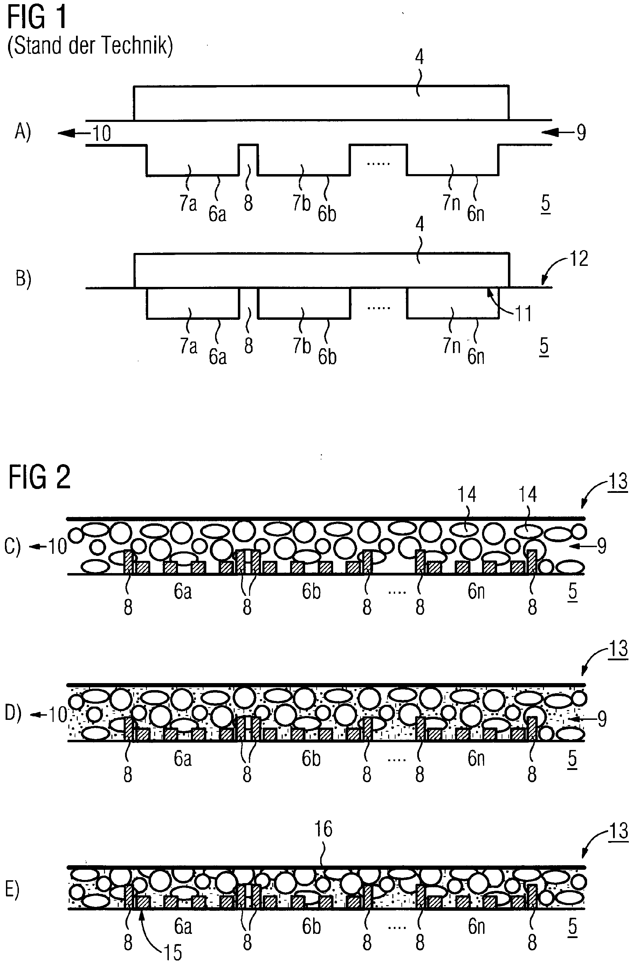

In Fig. 1 ist eine Einrichtung nach dem Stand der Technik, wie sie z.B. aus der Druckschrift DE 10058394 C1 bekannt ist, im offenen Zustand (Teilfigur A) und im geschlossenen Zustand (Teilfigur B) dargestellt. Die Einrichtung umfasst ein Sensor-Array 5, welches aus räumlich beabstandeten Sensoren 6a bis 6n aufgebaut ist. Die Sensoren 6a bis 6n sind in Form eines Arrays auf einer planaren Oberfläche angeordnet. Zwischen den Sensoren 6 ragen Wände 8 aus der planaren Oberfläche heraus. Die Wände 8 umgeben die Sensoren 6 vollständig, z.B. in Form von geschlossenen Ringen um jeden einzelnen Sensor 6a bis 6n. Parallel zur planaren Oberfläche ist in einem geringen Abstand, z.B. im Bereich von Mikrometer bis Zentimeter, ein steifer Stempel 4 angeordnet. Mit steifer Stempel 4 ist in diesem Zusammenhang ein Stempel gemeint, welcher aus einem inkompressiblen bzw. nur sehr wenig kompressiblen Material besteht. Im Zwischenraum zwischen dem steifen Stempel 4 und der planaren Oberfläche des Sensor-Arrays 5 mit Wänden 8 kann Flüssigkeit 28 fließen. Dazu können in der planaren Oberfläche benachbart zum Sensor-Array 5 Mikrokanäle ausgebildet sein, welche als Zu- 9 und Abfluss 10 dienen. Wird der Stempel 4 in Richtung planare Oberfläche des Sensor-Arrays 5 gedrückt, so kommt er in direkten mechanischen Kontakt mit den Wänden 8. Sind die Wände 8 so ausgebildet, dass sie gleich hoch aus der planaren Oberfläche ragen und jeden Sensor 6 vollständig umschließen bzw. umfassen, so steht der Stempel 4 bei paralleler Ausrichtung seiner planaren Stempeloberfläche zur planaren Oberfläche des Sensor-Arrays 5 mit allen Wänden 8 gleichzeitig in Kontakt und der Flüssigkeitsstrom ist vollständig unterbunden. Es sind Reaktionsräume 7a bis 7n gebildet, welche mit Flüssigkeit 28 gefüllt sind, und die begrenzt werden durch die planare Oberfläche, in welcher die Sensoren 6a bis 6n angeordnet sind, die Wände 8 und dem Stempel 4. Zwischen verschiedenen Reaktionsräumen 7a bis 7n findet kein Flüssigkeitsaustausch statt. Bei einer Messung mit den Sensoren 6a bis 6n nacheinander oder gleichzeitig, können unterschiedliche Reaktionen in unterschiedlichen Reaktionsräumen 7a bis 7n sich gegenseitig nicht beeinflussen. Ebenfalls kann der unterbundene Flüssigkeitsaustausch zwischen Reaktionsräumen 7a bis 7n zu einer Messung führen, mit geringerem Messfehler im Vergleich zu Messungen bei offenen Reaktionsräumen 7a bis 7n.

Ein Beispiel für Messungen mit Sensoren 6, bei welchen abgeschlossene Reaktionsräume 7a bis 7n zu einer Verkleinerung von Messfehlern führen, sind elektrochemische Messungen. Die Sensoren 6 im Fall von elektrochemischen Messungen bestehen beispielsweise aus Metallelektroden 15, z.B. fingerförmig auf der planaren Oberfläche aufgebrachten Goldelektroden. Diese können mit Fängermolekühlen beschichtet sein, spezifisch für die nachzuweisenden Moleküle in der Flüssigkeit 28. An die Sensoren 6, d.h. Goldelektroden, wird z.B. eine feste Spannung angelegt, und abhängig von an der Oberfläche bindenden Molekülen, ändert sich ein zu messender Stromfluss. Weitere bekannte elektrochemische Methoden sind zyklische Voltammetrie, Chronoamperometrie, Coulometrie, Impedanzspektroskopie, welche sich in den zu steuernden bzw. regelnden Messgrößen und/oder Steuer- bzw. Regelungsmethoden für Strom und Spannung unterscheiden. Den Methoden ist gemeinsam, dass die Messergebnisse stark davon abhängig sind, ob die zu vermessende Flüssigkeit 28 stationär oder als Strömung über den Sensoren 6 vorliegt. Eine einfache Messung, ohne störende Messsignale ist nur bei stationärer oder konstant strömender Flüssigkeit 28 möglich. Der einfachste, technisch zu realisierende Messaufbau besteht in der Realisierung einer stationären Flüssigkeit 28, wie sie z.B. in der in Teilfigur B der Fig. 1 gezeigten Einrichtung vorliegt.

Um jedoch eine stationäre Flüssigkeit 28 ohne Strömungen während einer Messung zu realisieren, müssen die Reaktionsräume 7a bis 7n vollständig voneinander getrennt sein. Dies ist nur der Fall, wenn der Stempel 4 gleichzeitig mit allen Wänden 8 in einem engen Kontakt steht, und zwischen keiner Wand und dem Stempel 4 ein Abstand vorliegt. Dies ist nur möglich, wenn alle Wände 8 mit der gleichen Höhe aus der planaren Oberfläche des Sensor-Arrays 5 herausragen. Eine solche Einrichtung setzt sehr aufwendige Herstellungsverfahren und eine genaue Kontrolle der Maße nach der Herstellung der Einrichtungen voraus.

In Fig. 2 ist eine Einrichtung entsprechend einem Ausführungsbeispiel der Erfindung in offenem/trockenem Zustand (Teilfigur C), in offenem/gefülltem Zustand (Teilfigur D) sowie in geschlossenem Zustand (Teilfigur E) dargestellt. Nicht näher ausgeführte Teile sind an sich, z.B. aus dem genannten Stand der Technik bekannt. Ein Sensor-Array 5, aufgebaut aus räumlich voneinander getrennten Sensoren 6a bis 6n, ist in einer planaren Oberfläche auf einem Substrat angeordnet. Die Sensoren 6 bestehen z.B. aus fingerförmig angeordneten Goldelektroden. In Fig. 2 ist ein Schnitt durch die Einrichtung, und somit durch die fingerförmigen Elektroden 15, dargestellt. Weitere Elektrodenmaterialien, welche verwendet werden können, sind Metalle wie z.B. Silber oder Silberchlorid-Paste, Palladium, Kupfer, Platin oder platiniertes Platin, Halbleitermaterialien, wie z.B. Titanoxid oder Silizium, Materialien wie z.B. Graphit, Glascarbon und Verbindungen der genannten Materialien.

Zwischen den Elektroden 15 sind Wände 8 angeordnet, welche die Elektroden 15 räumlich voneinander, zumindest in der planaren Ebene trennen. Die Wände 8 können in Form von flach auf der planaren Oberfläche liegenden geschlossenen Ringen ausgebildet sein, oder in Form von Rechtecksbegrenzungen (analog den Feldbegrenzungen eines Schachbrettes), oder in Form von Dreiecksbegrenzungen, oder in Form einer Wabenstruktur. Strukturen, welche wie ein Netz die Fläche möglichst vollständig überspannen, sind bevorzugt.

Die Wände 8 sind vorzugsweise aus dem gleichen Material ausgebildet wie das Trägersubstrat, auf welchem die Elektroden 15 angeordnet sind. Mögliche Materialien sind Silizium, Siliziumoxid, Plastik, Glas oder Photolack, z.B. PBO (Polybenzoxazol). Die Erfindung ist aber nicht auf diese Materialien beschränkt. Die Wände 8 können auch aus anderen Materialien als dem Trägermaterial bestehen.

Sie können aus der Oberfläche geätzt oder gestanzt sein, aufgedampft oder gesputtert sein, oder in Form von Photolack aufgebracht sein. Die Erfindung ist aber nicht auf Wände 8 beschränkt, welche nach den zuvor genannten Methoden hergestellt sind. Eine Vielzahl weiterer Methoden ist denkbar. Eine weitere Methode ist das Anordnen der Sensoren 6 in Vertiefungen in der Oberfläche, in einer Fläche parallel zur planaren Oberfläche. Die Wände 8 und das Trägermaterial sind bevorzugt nicht Flüssigkeitsdurchlässig.

Eine poröse Membran 13 ist formschlüssig auf der planaren Oberfläche des Sensor-Arrays 5 angeordnet. Unter Membran 13 ist in diesem Zusammenhang eine dünne Schicht zu verstehen, welche abhängig von äußeren Einflüssen wie z.B. Druck für Flüssigkeit 28 durchlässig (permeabel), halbdurchlässig (semipermeabel, nur für bestimmte Teile der Flüssigkeit 28 durchlässig, nicht für alle Substanzen in der Flüssigkeit 28 durchlässig) oder nicht durchlässig (impermeabel) sein kann. Poren in der Membran 13, welche miteinander verbunden sind, sorgen im entspannten Zustand für eine Durchlässigkeit gegenüber Flüssigkeiten 28. Unter entspanntem Zustand ist zu verstehen, dass auf die Membran 13 kein zusätzlicher Druck ausgeübt wird und die Membran 13 nicht komprimiert wird. Es wirkt auf die Membran 13 nur der Luftdruck. Ein Beispiel für ein Material, aus welchem die Membran 13 aufgebaut ist, ist Nitrocellulose. Weitere Möglichkeiten sind poröse, reversibel und/oder irreversibel komprimierbare Materialien wie z.B. behandelte Plastik- oder Gummi-Schichten bzw. Folien, Naturkautschuk, Schwämme oder Faserwerkstoffe, wie z.B. Baumwolle oder Glasfasern.

In der ersten Teilfigur C der Fig. 2 ist die poröse Membran 13 in einem entspannten Zustand gezeigt. Zwischen den Membranteilen 14, aus welcher die Membran 13 aufgebaut ist, sind Poren vorhanden. Diese Poren gehen ineinander über, so dass über die Poren ein Stofftransport, wie z.B. ein Gastrom oder ein Flüssigkeitsstrom, stattfinden kann. Typische Größenordnungen der Poren sind im Bereich von Nanometern bis Millimeter für den Durchmesser der Poren, insbesondere im Bereich von 0,1 bis 100 Mikrometer Durchmesser, besonders bevorzugt im Bereich von 1 bis 10 Mikrometer Durchmesser. In der Teilfigur C sind die Poren mit Luft gefüllt. Die Membran 13 ist formschlüssig auf der Oberfläche angebracht, d.h. zwischen der Membran 13 und der Oberfläche mit Wänden 8 und Elektroden 15 bestehen keine Hohlräume oder befinden sich keine anderen Stoffe. Die Membran 13 hat die inverse Form an ihrer der planaren Oberfläche zugewandten Seite wie die Form der Oberfläche mit darauf angeordneten Elektroden 15 und Wänden 8. Auf der der planaren Oberfläche abgewandten Seite der Membran 13 ist die in dem dargestellten Ausführungsbeispiel gezeigte Membran 13 eben, d.h. ohne mit aus einer planaren Oberfläche herausragenden Erhöhungen. Es sind aber auch andere Formen der Membran 13 möglich. Die Membran 13 ist vorzugsweise wie eine dünne, auf der Oberfläche formschlüssig aufgebrachte Folie ausgebildet.

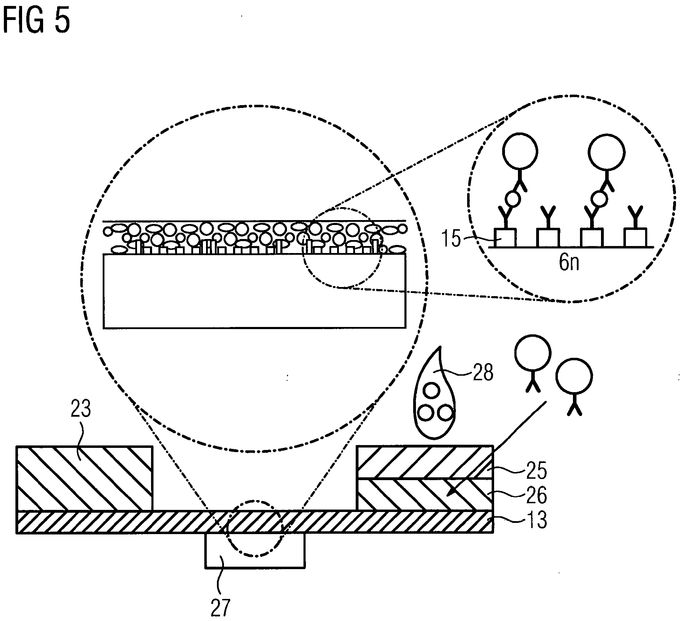

In der zweiten Teilfigur D der Fig. 2 ist die Membran 13 in einem entspannten Zustand, mit Flüssigkeit 28 befüllt gezeigt. Über einen Zu- 9 und Abfluss 10 kann Flüssigkeit 28 der Membran 13 zu bzw. aus der Membran 13 abgeführt werden. Der Zu- 9 und Abfluss 10 kann, wie in Fig. 2 gezeigt, parallel zur planaren Oberfläche oder wie in Fig. 5 senkrecht zur planaren Oberfläche erfolgen. Ein Flüssigkeitsstrom in der Membran 13, mit einer Strömungsrichtung bevorzugt parallel der planaren Oberfläche, ist durch die gesamte Membran 13 hindurch möglich. Die Membran 13 kann vollständig oder nur zum Teil mit Flüssigkeit 28 befüllt sein. Zum Teil bedeutet in diesem Zusammenhang, dass z.B. Lufteinschlüsse in der Membran 13 vorliegen. Vollständig befüllt bedeutet, dass die Poren zwischen den Membranteilen 14 vollständig mit Flüssigkeit 28 ausgefüllt sind. Das Befüllen der Membran 13 mit Flüssigkeit 28 kann unter anderem auf Grund von Druckunterschieden oder auf Grund von Kapillarkräften erfolgen.

In der dritten Teilfigur 3 der Fig. 2 ist die Membran 13 in einem zusammengedrückten, d.h. komprimierten Zustand gezeigt. Die Dicke der Membran 13 ist verkleinert durch einen Druck, welcher z.B. durch einen nicht gezeigten Stempel flächig auf die Membran 13 ausgeübt wird, senkrecht zur planaren Oberfläche. Eine andere Möglichkeit die Membrandicke zu reduzieren, ist das Ziehen der Membran 13 längs einer Richtung parallel zur planaren Oberfläche. In beiden Fällen verkleinert sich die Porengröße der Membran 13. Im Bereich senkrecht zur planaren Oberfläche über den Wänden 8 wird die Porengröße so stark verkleinert, dass die Poren vollständig verschwinden oder zumindest die Poren nicht mehr ineinander übergehen. Zwischen den Poren sind Membranteile 14 derart angeordnet, dass keine Verbindung mehr zwischen benachbarten Poren besteht. Ein Gas oder Flüssigkeitstransport in diesem Bereich ist vollständig oder zu einem großen Teil unterbunden. Eine elektrische Leitfähigkeit kann in diesem Bereich jedoch noch vorliegen, in einer sehr hochohmigen Form. Diese ist für hochohmige Potentialmessungen ausreichend, jedoch ungeeignet für die Ausbildung von lateralen elektrischen Strömen (parallel zur Sensor-Array 5 Oberfläche). Im Bereich senkrecht zur planaren Oberfläche über den Sensoren 6 sind die Poren nur verkleinert, jedoch stehen sie untereinander noch in direktem Kontakt. Ein Gas- oder Flüssigkeitstransport sowie elektrische Leitfähigkeit in diesem Bereich sind möglich. Die Flüssigkeit 28 kann so in diesem Bereich zu und von den Sensoren 6 weg transportiert werden.

Es entstehen im Bereich senkrecht zur planaren Oberfläche über den Sensoren 6 Reaktionsräume 7, in welchen ein Flüssigkeitstransport möglich ist. Diese sind durch die planare Oberfläche, auf welcher die Sensoren 6 angeordnet sind, durch die Wände 8, durch die Bereiche senkrecht zur planaren Oberfläche über den Wänden 8 mit verschlossenen Poren, und durch die Oberfläche der Membran 13 auf der der planaren Oberfläche gegenüberliegenden Seite 16, begrenzt. Zwischen benachbarten Reaktionsräumen 7 findet kein bzw. nur ein sehr stark eingeschränkter Flüssigkeitstransport statt. Dadurch können Reaktionen in den Reaktionsräumen 7 stattfinden, welche nicht oder nur sehr wenig von Reaktionen in benachbarten Reaktionsräumen 7 beeinflusst sind.

Im Fall der in der dritten Teilfigur E der Fig. 2 gezeigten, mit Flüssigkeit 28 gefüllten, zusammengedrückten (komprimierten) Membran 13, können Messungen an den Sensoren 6 mit geringerem Messfehler stattfinden als im Fall der unkomprimierten, mit Flüssigkeit 28 gefüllten Membran 13, welche in der zweiten Teilfigur D der Fig. 2 gezeigt ist. Strömungen parallel der planaren Oberfläche über mehrere verschiedene Sensoren 6a bis 6n hinweg sind unterbunden bzw. stark eingeschränkt im Fall der komprimierten Membran 13. Ein chemisches "Übersprechen", d.h. die Veränderung des Messergebnisses an einem Sensor 6 durch Reaktionen über einem anderen Sensor 6, sind unterbunden oder zumindest stark eingeschränkt.

Beispiele für Flüssigkeiten 28, die mit der dargestellten Einrichtung untersucht werden können, sind Körperflüssigkeiten wie Blut oder Urin oder Speichel, deren Bestandteile bzw. Reaktionsprodukte nach z.B. Aufschlussreaktionen nachgewiesen werden. Es können so z.B. DNA-Moleküle bzw. Fragmente mit Hilfe der Sensoren 6 nachgewiesen bzw. analysiert werden, Viren bzw. Antikörper, Peptide oder andere biochemischen Verbindungen können untersucht werden.

Als Flüssigkeiten 28 können aber auch Abwässer mit chemischen Verunreinigungen oder Trinkwasser untersucht werden bzw. andere chemische Substanzen in Flüssigkeiten 28 nachgewiesen werden.

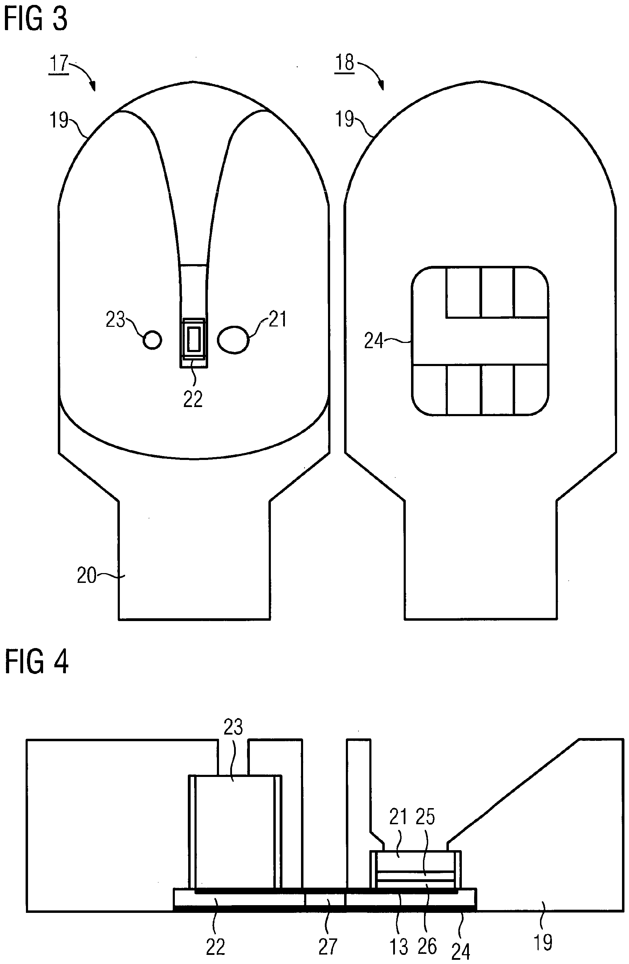

In den Fig. 3 bis 5 ist als eine mögliche Anwendung der erfindungsgemäßen Einrichtung und des erfindungsgemäßen Verfahrens ein Steckmodul für immunologische Schnelltests angedeutet.

Die Fig. 3 zeigt dabei die Vorder- 17 und Rückseite 18 des Steckmoduls, welches z.B. aus einem Plastik-Körper als Gehäuse 19 besteht, einem darin befindlichen Chip-Modul 22 mit Sensor-Array 5 auf einem Chip 27, elektrischen Kontakten 24, und einer auf dem Chip 27 befindlichen Membran 13. Das Steckmodul kann die in Fig. 3 dargestellte Form oder andere Formen, wie z.B. die einer Scheckkarte, aufweisen. In dem Plastikgehäuse 19 sind Kammern und Mikrokanäle für die Aufnahme, den Transport und Reaktionen sowie Abgabe der Flüssigkeit 28 ausgebildet. Ebenfalls Kammern für Reagenzien, wie z.B. Trockenreagenzien, welche für z.B. Aufschluss- und Nachweisreaktionen benötigt werden, können in dem Plastikgehäuse 19 realisiert sein.

Das Gehäuse 19 kann zur besseren Handhabung mit einem Griff 20 und mit Beschriftungen, wie z.B. Patienten- oder Probendaten ausgestattet sein. Alternativ können Daten auf dem Chip 27 gespeichert sein, welcher z.B. ein CMOS-Chip (Complementary Metal Oxide Semiconductor) ist, mit darauf befindlichem Sensor-Array 5 und im Chip 27 integrierter Datenverarbeitungs- und/oder Speichereinrichtung.

Dem Gehäuse 19 wird über einen Zufluss 21 im Gehäuse 19 Flüssigkeit 28 zugeführt. Die Flüssigkeit 28 wird durch die Membran 13 über den Chip 27 und das Sensor-Array 5 geleitet. Bevor die Messung beginnt, wird über einen Stempel auf die Membran 13 ein Druck ausgeübt, welcher während der Messung aufrechterhalten wird. Überschüssige Flüssigkeit 28 sowie Flüssigkeit 28 nach der Messung, wenn der Druck auf die Membran 13 aufgehoben ist, kann in einem Abfluss 23 mit Entlüftung entsorgt oder über diesen dem Gehäuse 19 entnommen werden.

Der Druck auf die Membran 13 während der Messung kann direkt über eine Öffnung im Gehäuse 19 auf die Membran 13 mit Hilfe eines Stempels ausgeübt werden, oder indirekt über das Gehäuse 19, welches dann auf die Membran 13 drückt. Der Stempel ist in einer nicht dargestellten Messapparatur, welche die Messelektronik umfasst, integriert.

Das Sensor-Array 5 wird über den Chip 27 und elektrische Kontakte 24, welche mit dem Chip 27 elektrisch verbunden sind und sich auf der Gehäuserückseite befinden, kontaktiert. Bei Einführung des Steckmoduls in die Messapparatur wird ein elektrischer Kontakt der Messapparatur mit den elektrischen Kontakten 24 des Steckmoduls hergestellt. Das Sensor-Array 5 kann über die Messelektronik der Messapparatur elektrisch angesprochen werden, und Messgrößen an die Messelektronik übermitteln, welche dann in der Messapparatur verarbeitet und ausgewertet werden. Ein Display oder andere optische sowie akustische Ausgabeeinrichtungen, welche an die Messapparatur angeschlossen sind, können das Messergebnis ausgeben.

In der Fig. 4 ist ein Schnitt durch das in der Fig. 3 gezeigte Steckmodul dargestellt. In dem Gehäuse 19 sind ein Enzym-Substrat-Pad 25, ein Konjugat-Pad 26, das Chip-Modul 22 mit einem Sensor-Array 5 auf z.B. einem CMOS-Chip 27 sowie die darauf angeordnete Membran 13 integriert. Über einen Flüssigkeits-Zufluss 21 kann die zu untersuchende Flüssigkeit 28, z.B. Blut 28, zugeführt werden. Wie in Fig. 5 in mehr Detail dargestellt ist, durchfließt die Flüssigkeit 28 beispielsweise ein Enzym-Substrat-Pad 25 und ein Konjugat-Pad 26. Es können auch weitere Substanz-Pads integriert sein, welche die zu untersuchende Flüssigkeit 28 mit chemischen Substanzen mischen und mit chemischen Reaktionen für die Untersuchung vorbereiten.

In dem in Fig. 5 dargestellten Beispiel wird das Blut aufgeschlossen, d.h. die Zellen geben Bestandteile wie z.B. DNA frei, welche wiederum in kleine Fragmente zerlegt werden. Anschließend werden die Fragmente an Marker, welche den Nachweis der Fragmente ermöglichen, angebunden.

Die so aufbereitete Flüssigkeit 28 wird durch die Membran 13 geleitet, d.h. über Kapillarkräfte und/oder einen äußeren Druck bzw. Gravitationskräfte wird die Flüssigkeit 28 von der Membran 13 aufgesaugt und/oder wird die Membran 13 mit der Flüssigkeit 28 befüllt. Die Membran 13 steht in direktem Kontakt mit dem Sensor-Array 5 bzw. ist auf diesem angeordnet. Die Flüssigkeit 28 wird so über das Sensor-Array 5 geleitet. Eine Öffnung im Gehäuse 19 über dem Chip 27 mit der darauf angeordneten Membran 13 ermöglicht die Beaufschlagung der Membran 13 mit einem Druck, z.B. durch einen Stempel, welcher auf die Membran 13 drückt. Der Druck ist so dimensioniert, dass in Bereichen mit Wänden 8 auf dem Sensor-Array 5 sich die Poren der porösen Membran 13 vollständig schließen, ohne dass die Wände 8 durch einen zu großen Druck beschädigt werden. In Bereichen direkt über den Sensoren 6 sind die Poren verkleinert im Vergleich zu ihrer ursprünglichen Größe vor Beaufschlagung der Membran 13 mit Druck. Sie sind aber im Bereich direkt über den Sensoren 6 nicht vollständig geschlossen, wie es im Bereich über den Wänden 8 der Fall ist. Es entstehen in der mit Flüssigkeit 28 befüllten Membran 13 voneinander getrennte, abgeschlossene Reaktionsräume 7, in welchen Reaktionen ablaufen können. Sensoren 6 in den Reaktionsräumen 7 können unbeeinflusst von Reaktionen in benachbarten Reaktionsräumen 7 Reaktionsprodukte registrieren und nachweisen. Flüssigkeitsströmungen über mehrere Reaktionsräume 7 hinweg sind unterbunden und führen zu keinen Störsignalen bei der Messung bzw. zu Messfehlern.

Nach Ablauf der Reaktionen und den Messungen mit Hilfe der Sensoren 6 wird der Druck auf die Membran 13 aufgehoben, und die Flüssigkeit 28 kann in einen Flüssigkeits-Abfluss 23 fließen. Sie kann dort verbleiben oder für weitere Untersuchungen entnommen werden. Der Flüssigkeits-Abfluss 23 dient ebenfalls zur Entsorgung von überschüssiger Flüssigkeitsmenge vor den Messungen und für die Entsorgung von z.B. Spülflüssigkeiten, welche vor und nach Messungen zum Spülen der Membran 13 verwendet wird.

Das Steckmodul kann für die einmalige Verwendung ausgelegt sein, d.h. als Einwegmodul, oder für die mehrmalige Verwendung.

## Patentansprüche

1. Einrichtung zum Nachweis von Flüssigkeiten (28) oder Substanzen aus Flüssigkeiten (28) in räumlich verschiedenen Reaktionsbereichen,
mit einem Sensor-Array (5), welches aus wenigstens zwei Sensoren (6) besteht, und
mit wenigstens einer Membran (13), welche auf dem Sensor-Array (5) angeordnet ist,
wobei die wenigstens eine Membran (13) permeable oder semipermeable, poröse Eigenschaften aufweist,
wobei die wenigstens zwei Sensoren (6) planar auf einer Oberfläche angeordnet sind,
**dadurch gekennzeichnet,**
**dass** die wenigstens zwei Sensoren (6) durch aus der Oberfläche herausragende Seitenwände (8), die nicht Teil der Membran sind, räumlich voneinander vollständig oder zumindest teilweise getrennt sind, und
**dass** die Membran (13) durch eine Druckkraft, welche flächig auf eine Hauptoberfläche der Membran (13) ausübbar ist, mechanisch verformbar ist, und dazu ausgebildet ist, durch das mechanische Verformen einen Flüssigkeitsaustausch zwischen unterschiedlichen Bereichen der Membran (13), welche benachbart zu den räumlich getrennten Sensoren (6) angeordnet sind, vollständig zu unterbinden.

2. Einrichtung nach Anspruch 1, **dadurch gekennzeichnet, dass** die Permeabilität der Membran (13) abhängig von einer Zug- und/oder Druckkraft einstellbar ist und/oder ein Aktuator zur Ausübung von Druck über der wenigstens einen Membran (13) angeordnet ist, insbesondere ein Aktuator in Form eines Stempels (4).

3. Einrichtung nach einem der vorhergehenden Ansprüche, **dadurch gekennzeichnet, dass** die Membran (13) aus Nitrocellulose besteht oder Nitrocellulose enthält.

4. Einrichtung nach einem der vorhergehenden Ansprüche, **dadurch gekennzeichnet, dass** die Membran (13) eine Porengröße im Bereich von 0,1 bis 100 Mikrometer Durchmesser, insbesondere im Bereich von 1 bis 10 Mikrometer Durchmesser aufweist.

5. Einrichtung nach einem der vorhergehenden Ansprüche, **dadurch gekennzeichnet, dass** das Sensor-Array (5) elektrochemische Sensoren (6) mit Elektroden (15), insbesondere Mikro-Elektroden, enthält oder daraus aufgebaut ist und/oder die Poren der wenigstens einen Membran (13) einen Durchmesser aufweisen, welcher größer ist als der Abstand zwischen wenigstens zwei benachbarten Elektroden (15) und/oder die Elektroden die Form von ineinander greifenden kammförmigen Fingern mit einer Höhe im Bereich von 0,1 bis 1 Mikrometer, einer Breite im Bereich von 1 bis 10 Mikrometern und einem Abstand von benachbarten Elektroden (15) im Bereich von 1 bis 10 Mikrometern aufweisen.

6. Einrichtung nach einem der vorhergehenden Ansprüche, **dadurch gekennzeichnet, dass** die Seitenwände (8) eine Höhe im Bereich von 1 bis 10 Mikrometern und eine Breite im Bereich von 1 bis 100 Mikrometern aufweisen.

7. Einrichtung nach einem der vorhergehenden Ansprüche, **dadurch gekennzeichnet, dass** die Seitenwände (8) ringförmig um jeden Sensor (6) herum angeordnet sind, insbesondere in Form geschlossener Ringe, welche insbesondere einen Durchmesser der umschlossenen Fläche im Bereich von 150 bis 200 Mikrometern aufweisen.

8. Einrichtung nach einem der vorhergehenden Ansprüche, **dadurch gekennzeichnet, dass** die Membran (13) formschlüssig auf der Oberfläche angeordnet ist, insbesondere planar ohne Knicke in der Membran (13).

9. Verfahren zum Nachweis von Flüssigkeiten (28) oder Substanzen aus Flüssigkeiten (28) in räumlich verschiedenen Reaktionsbereichen, unter Verwendung der Einrichtung nach einem der vorhergehenden Ansprüche, mit den Schritten
a) Befüllen wenigstens einer permeablen oder semipermeablen Membran (13), insbesondere der Poren (6) dieser Membran (13), welche auf einem Sensor-Array (5) angeordnet ist, mit einer zu analysierenden Flüssigkeit (28), wobei Sensoren (6) des Sensor-Arrays (5) planar auf einer Oberfläche angeordnet und durch Seitenwände (8) räumlich voneinander vollständig oder zumindest teilweise getrennt sind,
b) Mechanisches Verformen der Membran (13) derart, dass deren Permeabilität eingestellt wird, wobei die mechanische Verformung der Membran (13) durch eine Druckkraft erfolgt, welche flächig auf eine Hauptoberfläche der Membran (13) ausgeübt wird,
c) Messen von Wechselwirkungen der zu analysierenden Flüssigkeit (28) oder von Substanzen aus der Flüssigkeit (28) mit Sensoren (6) eines Sensor-Arrays (5).

10. Verfahren nach Anspruch 9, **dadurch gekennzeichnet, dass** mindestens zwei der Schritte a) bis c) gleichzeitig oder in der Reihenfolge a) bis c) nacheinander durchgeführt werden.

11. Verfahren nach einem der Ansprüche 9 oder 10, **dadurch gekennzeichnet, dass** die Ausübung der Druckkraft mit einem Stempel (4) erfolgt.

12. Verfahren nach einem der Ansprüche 9 bis 11, **dadurch gekennzeichnet, dass** die Druckkraft zu einem Zusammenpressen der Membran (13) führt.

13. Verfahren nach einem der Ansprüche 9 bis 12, **dadurch gekennzeichnet, dass** durch das mechanische Verformen der Membran (13) eine teilweise oder vollständige Unterbindung eines Flüssigkeitsaustausches zwischen unterschiedlichen Bereichen der Membran (13) erfolgt, welche benachbart zu räumlich getrennten Sensoren (6) angeordnet sind.

14. Verfahren nach Anspruch 13, **dadurch gekennzeichnet, dass** die vollständige Unterbindung eines Flüssigkeitsaustausches zwischen unterschiedlichen Bereichen der Membran (13) durch die Seitenwände (8), insbesondere ringförmige Seitenwände (8) um die Sensoren (6) angeordnet, erfolgt, wobei die Seitenwände (8) bei Druckeinwirkung auf die Membran (13) im Bereich der Seitenwände (8) zu einer kleineren Porengröße der Membran (13) im Vergleich zur Porengröße im Bereich der Sensoren (6) führen.

15. Verfahren nach einem der Ansprüche 9 bis 14, **dadurch gekennzeichnet, dass** das Befüllen der Membran (13) mit zu analysierender Flüssigkeit (28) durch Vollsaugen der Membran (13) mit der Flüssigkeit aufgrund von Kapillarkräften erfolgt und/oder durch Druckunterschiede innerhalb der Flüssigkeit (28).

16. Verfahren nach einem der Ansprüche 9 bis 15, **dadurch gekennzeichnet, dass** das Befüllen der Membran (13) mit zu analysierender Flüssigkeit (28) durch Verdrängen eines Gases aus der Membran (13) durch die Flüssigkeit (28) erfolgt und/oder durch Strömen, insbesondere kontinuierliches Strömen, der Flüssigkeit (28) durch die Membran (13) erfolgt, insbesondere auf Grund einer Druckdifferenz oder auf Grund von Kapillarkräften.

17. Verfahren nach einem der Ansprüche 9 bis 16, **dadurch gekennzeichnet, dass** das Messen von Wechselwirkungen der zu analysierenden Flüssigkeit (28) oder von Substanzen aus der Flüssigkeit (28) mit Sensoren (6) des Sensor-Arrays (5) über elektrochemische Messungen, insbesondere Voltammetrische- und/oder Amperometrische- und/oder Coulometrische- und/oder Impedanz-Messungen und/oder über Redoxcycling, erfolgt.

18. Verfahren nach einem der Ansprüche 9 bis 17, **dadurch gekennzeichnet, dass** während der Messung von Wechselwirkungen der zu analysierenden Flüssigkeit (28) oder von Substanzen aus der Flüssigkeit (28) mit Sensoren (6) des Sensor-Arrays (5) zwischen räumlich verschiedenen Reaktionsbereichen, welche an die Sensoren (6) direkt angrenzen, kein die Messung störender Flüssigkeitsaustausch und/oder Substanzaustausch, insbesondere überhaupt kein Flüssigkeitsaustausch und/oder Substanzaustausch, stattfindet.

## Claims

1. Apparatus for the detection of liquids (28) or substances from liquids (28) in spatially different reaction regions,
having a sensor array (5) consisting of at least two sensors (6), and
having at least one membrane (13) arranged on the sensor array (5),
wherein the at least one membrane (13) has permeable or semipermeable, porous properties,
wherein the at least two sensors (6) are arranged on a surface in a planar manner, **characterised**
**in that** the at least two sensors (6) are completely or at least partially spatially separated from each other by side walls (8) which protrude from the surface and are not part of the membrane, and
**in that** the membrane (13) is mechanically deformable by a compressive force, which can be exerted in a planar manner on a main surface of the membrane (13), and is designed to completely prevent, by means of the mechanical deformation, a liquid exchange between different regions of the membrane (13), which are arranged adjacent to the spatially separated sensors (6).

2. Apparatus according to claim 1, **characterised in that** the permeability of the membrane (13) is adjustable depending on a tensile and/or compressive force and/or an actuator is arranged above the at least one membrane (13) to exert pressure, particularly an actuator in the form of a stamp (4).

3. Apparatus according to any one of the preceding claims, **characterised in that** the membrane (13) consists of nitrocellulose or contains nitrocellulose.

4. Apparatus according to any one of the preceding claims, **characterised in that** the membrane (13) has a pore size in the range of 0.1 to 100 micrometres in diameter, particularly in the range of 1 to 10 micrometres in diameter.

5. Apparatus according to one of the preceding claims, **characterised in that** the sensor array (5) contains electrochemical sensors (6) with electrodes (15), particularly micro-electrodes, or is constructed therefrom, and/or the pores of the at least one membrane (13) have a diameter which is greater than the distance between at least two adjacent electrodes (15) and/or the electrodes have the form of interlocking, comb-shaped fingers with a height in the range of 0.1 to 1 micrometre, a width in the range of 1 to 10 micrometres and a distance between adjacent electrodes (15) in the range of 1 to 10 micrometres.

6. Apparatus according to any one of the preceding claims, **characterised in that** the side walls (8) have a height in the range of 1 to 10 micrometres and a width in the range of 1 to 100 micrometres.

7. Apparatus according to any one of the preceding claims, **characterised in that** the side walls (8) are arranged in an annular manner around each sensor (6), particularly in the form of closed rings, which have particularly a diameter of the enclosed surface in the range of 150 to 200 micrometres.

8. Apparatus according to any one of the preceding claims, **characterised in that** the membrane (13) is arranged in a form-fit manner on the surface, particularly in a planar manner without kinks in the membrane (13).

9. Method for the detection of liquids (28) or substances from liquids (28) in spatially different reaction regions, using the apparatus according to one of the preceding claims, comprising the steps:
a) filling at least one permeable or semipermeable membrane (13), particularly the pores (6) of this membrane (13), which is arranged on a sensor array (5), with a liquid (28) to be analysed, wherein sensors (6) of the sensor array (5) are arranged in a planar manner on a surface and are completely or at least partially spatially separated from each other by side walls (8),
b) mechanically deforming the membrane (13) such that its permeability is adjusted, wherein the mechanical deformation of the membrane (13) occurs by means of a compressive force which is exerted in a planar manner on a main surface of the membrane (13),
c) measuring interactions of the liquid (28) to be analysed or of substances from the liquid (28) with sensors (6) of a sensor array (5).

10. Method according to claim 9, **characterised in that** at least two of steps a) to c) are performed simultaneously or successively in the order of a) to c).

11. Method according to any one of claims 9 or 10, **characterised in that** the exertion of the compressive force occurs by means of a stamp (4).

12. Method according to any one of claims 9 to 11, **characterised in that** the compressive force leads to a compression of the membrane (13).

13. Method according to any one of claims 9 to 12, **characterised in that**, by means of the mechanical deformation of the membrane (13), a partial or complete prevention of a liquid exchange between different regions of the membrane (13), which are arranged adjacent to spatially separated sensors (6) occurs.

14. Method according to claim 13, **characterised in that** the complete prevention of a liquid exchange between different regions of the membrane (13) occurs by means of the side walls (8), particularly annular side walls (8) arranged around the sensors (6), wherein the side walls (8), upon application of pressure on the membrane (13) in the region of the side walls (8), lead to a smaller pore size of the membrane (13) compared with the pore size in the region of the sensors (6).

15. Method according to any one of claims 9 to 14, **characterised in that** the filling of the membrane (13) with liquid (28) to be analysed occurs by sucking the membrane (13) full of the liquid by virtue of capillary forces and/or by pressure differentials within the liquid (28).

16. Method according to any one of claims 9 to 15, **characterised in that** the filling of the membrane (13) with liquid (28) to be analysed occurs by displacing a gas from the membrane (13) by the liquid (28) and/or by flows, particularly continuous flows, of the liquid (28) through the membrane (13), particularly by virtue of a pressure differential or capillary forces.

17. Method according to any one of claims 9 to 16, **characterised in that** the measurement of interactions of the liquid (28) to be analysed or of substances from the liquid (28) with sensors (6) of the sensor array (5) occurs by means of electrochemical measurements, particularly voltammetric and/or amperometric and/or coulometric and/or impedance measurements and/or by redox cycling.

18. Method according to any one of claims 9 to 17, **characterised in that** during the measurement of interactions of the liquid (28) to be analysed or of substances from the liquid (28) with sensors (6) of the sensor array (5) between spatially different reaction regions, which directly adjoin the sensors (6), no liquid exchange and/or substance exchange disturbing the measurement takes place, particularly no liquid exchange and/or substance exchange at all.

## Revendications

1. Dispositif de détection de liquides (28) ou de substances provenant de liquides (28) dans des zones de réaction spatialement différentes,
comprenant un ensemble de capteurs (5), qui est constitué d'au moins deux capteurs (6), et
comprenant au moins une membrane (13) qui est disposée sur l'ensemble de capteurs (5),
ladite au moins une membrane (13) présentant des propriétés perméables, semi-perméables ou poreuses,
lesdits au moins deux capteurs (6) étant disposés à plat sur une surface, **caractérisé**
**en ce que** lesdits au moins deux capteurs (6) sont spatialement séparés l'un de l'autre entièrement ou au partiellement par des parois latérales (8), faisant saillie de la surface, qui ne font pas partie de la membrane, et
**en ce que** la membrane (13) est déformable mécaniquement par une force de pression qui peut être exercée à plat sur une surface principale de la membrane (13) et est conçue pour empêcher complètement, par la déformation mécanique, un échange de liquide entre les différentes zones de la membrane (13), qui sont disposées adjacentes aux capteurs (6) spatialement séparés.

2. Dispositif selon la revendication 1, **caractérisé en ce que** la perméabilité de la membrane (13) est réglable en fonction d'une force de pression et/ou de traction et/ou **en ce qu'**un actionneur est disposé sur ladite au moins une membrane (13) pour exercer la pression, en particulier un actionneur conçu sous la forme d'un poinçon (4).

3. Dispositif selon l'une des revendications précédentes, **caractérisé en ce que** la membrane (13) est constituée de nitrocellulose ou elle contient de la nitrocellulose.

4. Dispositif selon l'une des revendications précédentes, **caractérisé en ce que** la membrane (13) présente un diamètre de pore dans la plage comprise entre 0,1 et 100 micromètres, en particulier dans une plage comprise entre 1 et 10 micromètres.

5. Dispositif selon l'une des revendications précédentes, **caractérisé en ce que** l'ensemble de capteurs (5) contient ou est composé de capteurs électrochimiques (6) dotés d'électrodes (15), en particulier de micro-électrodes, et/ou les pores de ladite au moins une membrane (13) présentent un diamètre qui est supérieur à la distance entre au moins deux électrodes adjacentes (15) et/ou les électrodes sont conçues sous la forme de doigts en forme de dents s'engrenant les unes dans les autres d'une hauteur dans la plage comprise entre 0,1 et 1 micromètre, d'une largeur dans la plage comprise entre 1 et 10 micromètres et une distance entre les électrodes adjacentes (15) étant dans la plage comprise entre 1 et 10 micromètres.

6. Dispositif selon l'une des revendications précédentes, **caractérisé en ce que** les parois latérales (8) présentent une hauteur dans la plage comprise entre 1 et 10 micromètres et une largeur dans une plage comprise entre 1 et 100 micromètres.

7. Dispositif selon l'une des revendications précédentes, **caractérisé en ce que** les parois latérales (8) sont disposées en cercle autour de chaque capteur (6), en particulier sous la forme d'anneaux fermés, qui présentent en particulier un diamètre de la surface fermée dans la plage comprise entre 150 et 200 micromètres.

8. Dispositif selon l'une des revendications précédentes, **caractérisé en ce que** la membrane (13) est disposée par complémentarité de forme sur la surface, en particulier à plat sans plis dans la membrane (13).

9. Procédé de détection de liquides (28) ou de substances provenant de liquides (28) dans des zones de réaction spatialement différentes, faisant appel au dispositif selon l'une des revendications précédentes, comprenant les étapes consistant à
a) remplir au moins une membrane (13) perméable ou semi-perméable, en particulier les pores (6) de ladite membrane (13) qui est disposée sur un ensemble de capteurs (5), d'un liquide (28) à analyser, les capteurs (6) de l'ensemble de capteurs (5) étant disposés à plat sur une surface et étant spatialement séparés les uns des autres, complètement ou au moins partiellement par des parois latérales (8),
b) déformer mécaniquement la membrane (13) de telle sorte à régler sa perméabilité, la déformation mécanique de la membrane (13) s'effectuant par une force de pression qui est exercée à plat sur une surface principale de la membrane (13),
c) mesurer les interactions du liquide (28) à analyser ou des substances provenant du liquide (28) avec les de capteurs (6) d'un ensemble de capteurs (5).

10. Procédé selon la revendication 9, **caractérisé en ce qu'**au moins deux des étapes a) à c) s'effectuent simultanément ou successivement dans l'ordre a) à c).

11. Procédé selon l'une des revendications 9 ou 10, **caractérisé en ce que** l'exercice de la force de pression s'effectue avec un poinçon (4).

12. Procédé selon l'une des revendications 9 à 11, **caractérisé en ce que** la force de pression permet d'obtenir une compression de la membrane (13).

13. Procédé selon l'une des revendications 9 à 12, **caractérisé en ce que** la déformation mécanique de la membrane (13) empêche partiellement ou complètement un échange de liquide entre les différentes zones de la membrane (13) qui sont disposées adjacentes aux capteurs (6) spatialement séparés.

14. Procédé selon la revendication 13, **caractérisé en ce que** la suppression totale d'un échange de liquide entre les différentes zones de la membrane (13) s'effectue par les parois latérales (8), en particulier des parois latérales (8) annulaires disposées autour des capteurs (6), les parois latérales (8) permettent d'obtenir par effet de pression sur la membrane (13) dans la zone des parois latérales (8) une plus petite taille de pore de la membrane (13) par rapport à la taille de pore dans la zone des capteurs (6).

15. Procédé selon l'une des revendications 9 à 14, **caractérisé en ce que** le remplissage de la membrane (13) de liquide à analyser (28) s'effectue par imprégnation de la membrane (13) de liquide dû aux forces capillaires et/ou par différence de pression à l'intérieur du liquide (28).

16. Procédé selon l'une quelconque des revendications 9 à 15, **caractérisé en ce que** le remplissage de la membrane (13) de liquide à analyser (28) s'effectue par déplacement d'un gaz hors de la membrane (13) par le liquide (28) et/ou par écoulement, en particulier écoulement continu, du liquide (28) à travers la membrane (13), en particulier dû à une différence de pression ou dû à des forces capillaires.

17. Procédé selon l'une des revendications 9 à 16, **caractérisé en ce que** la mesure des interactions du liquide (28) à analyser ou des substances provenant du liquide (28) avec les capteurs (6) de l'ensemble de capteurs (5) s'effectue par mesures électrochimiques, en particulier par mesures voltamétriques et/ou ampérométriques et/ou coulométriques et/ou d'impédance et/ou par cycle oxydo-réduction.

18. Procédé selon l'une des revendications 9 à 17, **caractérisé en ce que** pendant la mesure des interactions du liquide (28) à analyser ou des substances provenant du liquide (28) avec les capteurs (6) de l'ensemble de capteurs (5) entre les zones de réaction spatialement différentes, qui sont directement adjacentes aux capteurs (6), ne se produit aucun échange de substance et/ou aucun échange de liquide qui perturbe la mesure, en particulier absolument aucun échange de liquide et/ou aucun échange de substance.
